# EUROPEAN PATENT SPECIFICATION

(11) **EP 1 041 880 B1**
(45) Date of publication and mention of the grant of the patent: **27.01.2010**
(21) Application number: 98946099.3
(22) Date of filing: 17.09.1998
(51) Int. Cl.: A01N 37/20, A61K 31/198, A61P 43/00

(54) **ADMINISTRATION OF ARGININE TO WARM COOL/COLD TISSUE**
VERABREICHUNG VON ARGININ ZUR ERWÄRMUNG VON KALTEM/KÜHLEM GEWEBE
ADMINISTRATION D'ARGININE AFIN DE CHAUFFER DES TISSUES FROIDS

(30) Priority: 17.09.1997 US 932227; 17.09.1997 US 932595; 17.09.1997 US 936188; 17.09.1997 US 936189
(43) Date of publication of application: 11.10.2000
(60) Divisional application: 09014985.7
(73) Proprietor: Strategic Science & Technologies, LLC, Cambridge MA 02141 (US)
(72) Inventor: FOSSEL, Eric, South Hero, VT 05486 (US)
(74) Representative: Torggler, Paul Norbert
(86) International application number: PCT/US1998/019429
(87) International publication number: WO 1999/013717

(56) References cited:
- WO-A-95/13060
- WO-A-97/16983
- FR-A- 2 602 678
- FR-M- 5 940
- US-A- 5 595 753
- US-A- 5 629 002
- PATENT ABSTRACTS OF JAPAN vol. 016, no. 148 (C-0928), 13 April 1992 (1992-04-13) & JP 04 005231 A (MORISHITA PHARMACEUT CO LTD; others: 01), 9 January 1992 (1992-01-09)

## Description

### Background of the invention

### Field of the invention

This invention relates, in general, to a delivery vehicle, either topical or oral which contains substances including arginine. L-arginine and alkyl esters of L-arginine. The purpose of this delivery vehicle is to introduce arginine or L-arginine into human or mammalian tissue for the purpose producing beneficial effects such as the warming of cold or cool tissues.

### Prior Art

Approaches to improving local blood flow have been many and consist of both systemic and topical approaches. Many beneficial effects could be obtained should improvement in local blood flow be achieved since impairment of local blood flow causes a variety of negative consequences. Among these are cold hands and feet, certain forms of impotence, baldness, and leg ulcers.

The fundamental basis for cold tissue of the hands, fingers, feet and toes as well as other cold tissue is insufficient blood to the tissue. it has been suggested by some that increasing blood flow through relaxation of blood vessels, particularly small a very small vessels will warm cold tissue. However, many attempts to warm by use of agents which produce vasodilation and therefore increased blood flow have produced negative resuits.

Previously cold hands or feet have been treated by wearing warm seeks or gloves, sometimes even socks or gloves which are mechanically heated. The use of hot packs and glove or shoe inserts which generate heat through chemical reactions have also been potential solutions, Another method of treatment is the application of certain liniments which are irritants. These liniments include the red pepper derived substance, capsicum, and its source extract oleoresin capsicum. More recently, topical creams containing nitroglycerin have been used. However, nitroglycerin is acardioactive drug, and thus its use raises concerns about its effect on the heart. Although all of these approaches may work at one level or another, they are often extremely transient in nature. US 5,595,753 discloses topical formulations and methods for treating haemorrhoidal pain and sphincter and the muscle spasm in the gastrointestinal tract US 5,629,002 discloses cosmetic or pharmaceutic preparations for improving hair quality and stimulating growth of the hair, the preparations comprising L-arginine. WO 9513060 discloses that L-arginine increases blood how and can therefore be used to formulate topical applications for increasing hair growth

### SUMMARY OF THE INVENTION

In accordance with the present invention, it has been discovered that the delivery of the nitric oxide precursor, arginine, L-arginine salts and alkyl esters of L-arginine (ethyl, methyl, propyl, isopropyl, butyl-isobutyl, t-butyl) by a topical approach produces a variety of beneficial effects due to the increased blood flow caused by the subsequent release of nitric oxide into the blood in the presence of a delivery carrier comprising a concentration of ionic salt, wherein the ionic salt has an ionic strength greater than two times the physiological ionic strength of blood, These beneficial effects include the warming of cool or cold tissue.

In one important embodiment of the present invention, when a delivery vehicle containing arginine or arginine derivatives in a concentration sufficient to produce the desired effects, along with sodium chloride or other salts at a concentration sufficient to produce a hostile biophysical environment, in either a topical form applied to a selected area of cool or cold tissue, the tissue is subsequently warmed. The warming of the tissue is caused by the increase in blood flow to the treated area. This warming effect can be prolonged, often lasting from 2-18 hours. In persons with very cold tissue (for example 22°C) this warming effect can have a magnitude of 10°C more.

Another embodiment of the present invention is the application of a delivery vehicle containing arginine or arginine derivative in a concentration sufficient to produce the desired effects, along with sodium chloride or other salts at a concentration sufficient to produce a hostile biophysical environment, in a topical form,

Another embodiment in accordance with the present invention is the application of a delivery vehicle containing arginine or arginine derivatives in a concentration sufficient to produce the desired effects, along with sodium chloride or other salts at a concentration sufficient to produce a hostile biophysical environment and capsaicin or oleoresin capsicum in concentrations sufficient to produce the desired effect, in a topical form.

### OBJECTS OF THE PRESENT INVENTION

Accordingly, a primary object of the present invention is to prevent mammalian or human tissue from becoming cold prior to entering into situations which induce cold hands and feet such as skiing or other winter outdoors activities by increasing blood flow to a selected area or areas of the body through the use of a nitric oxide releasing substance.

### DETAILED DESCRIPTION OF PREFERRED EMBODIMENTS

At the outset, it is to be understood that the invention is described in its broadest overall aspects with a more detailed description following. It is envisaged to deliver arginine or its derivatives to cause beneficial effects by its release of nitric oxide. The present invention also relies on the discovery that a carrier or vehicle for arginine will expel the arginine if it contains, in addition to the arginine, an agent which causes the arginine to leave the carrier and enter the tissue,

### THE TOPICAL DELIVERY VEHICLE

One embodiment of the present invention comprises a topical delivery vehicle having properties of excellent absorption into the skin. This topical delivery vehicle contains L-arginine hydrochloride (12.5% w/v), choline chloride (10%), sodium chloride (5% w/v)and magnesium chloride (5% w/v). As used herein, all expressions of concentration using the designation"%wlv" shall mean percent weight per total volume of the preparation regardless of form, e.g., cream, tablet, liquid, unless specified otherwise.

The components of the base cream may be those commonly found in hand creams, such as water(20-80%w/v), mineral oil(3-18%w/v), glyceryl stearatc(0.5-12%wlv), squalene(0.2-12%w/v), cetyl alcohol(0.1-11%w/v), propylene glycol stearate(0:1-11 %w/v), wheat germ oil (0.1-6%w/v), glyceryl stearate(0.1-6%wlv), isopropyl myristatl(0,1-6°%W/V) stearyl stearate(0.1 - 6%w/v), polysorbate 60(0.1-5%w/v), propyleneglycol (0.0-5%w/v), tocopherol acetate (0.05-5%), collagen (0.05-5%), sorbitan stearate (0.055%), vitamin A & D(0.02-4%w/v), triethanolamine <RTI (0.01-4%w/v), methylparaben(0.01-4%W/V) aloe vera extract(0.01-4%w/v), imidazolidinyl urea(0.01-4%w/v), propylparaben(0.01-4%w/v), bha(0.01-4%w/v), L-arginine hydrocholide (0,25% to25%w/v), sodium chloride (0.25% to25%w/v), magnesium chloride (0.25% to25%w/v).

L-arginine hydrochloride provides a precursor to the molecule, nitric oxide,NO Nitric oxide is the substance that relaxes the blood vessels, allowing for increased blood flow The concentration of the L-arginine-based compound, e.g., L-arginine hydrochloride is preferably about between 0.25to 25%w/v.

Choline chloride, sodium chloride and magnesium chloride are nonlimiting examples of salts which provide a high ionic strength environment for the highly charged molecule L-arginine. This high ionic strength environment is an example of a hostile biophysical environment for L-arginine. That is, the highly charged ionic strength provided by the salts to the L-arginine carrier is an unfavorable environment for the highly charged L-arginine which facilitates or promotes L-arginine migration out of the carrier and into a more hospitable, less charged environment such as human tissue. The ionic strength is preferably any amount greater than two times the physiological ionic strength of blood,

### OTHER ACTIVE INGREDIENTS

While L-arginine hydrochloride is the preferred active agent for use as a nitric oxide releasing substance other agents could be used which are also precursors or donors of nitric oxide. Specifically, L-arginine hydrochloride is preferred due to the fact that it is a natural occurring compound that is nontoxic highly soluble and inexpensive. Other precursors which may be used include D,L -argirline, L-arginine, alkyl (ethyl, methyl, propyl, isopropyl, butyl, isobutyl, t-butyl) ester of L-arginine and salts thereof.
Pharmaceutically acceptable salts include hydrochloride, glutamate, butyrate, and glycolate.

In the case of an alternative active agent were used it would be simply substituted for L-arginine in a delivery preparation and the preparation used as in the case of the L-arginine preparation. The cream may contain capsaicin or oleoresin capsicum in addition to L-arginine.

### OTHER MEANS OF EFFECTING ABSORPTION

A variety of carriers for effecting absorption are possible. One approach to effectuate the absorption of a highly charged molecule such as L-arginine into tissue is to either create a biophysically hostile environment in the delivery vehicle such that L-arginine would prefer to be in tissue. Other approaches include packaging L-arginine in such a way that it is carried into tissue and/or neutralize its charge by derivatization or forming a neutral salt Examples of biophysically hostile environments, include but are not limited to, high ionic strength, high or low pHandtor highly hydrophobic environments. Examples of packaging which would be carried into tissue include liposomes or emulsions of collagen, collagen peptides or other components of skin or basement membrane. An example of neutralization of charge include the salt, arginine glutamate which is electronically neutral. Other acceptable arginine salts are set forth herein above.

In each case of creating a hostile biophysical environment for the active agent, the agent was added to an appropriate preparation. In the case of creating a high ionic strengt ion environment, salts such as but not limited to sodium chloride, potassium chloride, choline chloride, lithium chloride, alone or in combination were added in high concentration to achieve an ionic strength greater than two times the physiological strength of blood. Other highly charged molecules such as polylysine, polyglutamine, polyaspartate or copolymers of such charged amino acids may be used to create the hostile biophysical environment.

Alternatively a hostile biophysical environment may be created by placing the highly charged L-arginine in an hydrophobic, oily environment such as in an oil-based cream containing little or no water. The preferred hydrophobicity or rho (p) is greater than two times the physiological hydrophobicity of blood. Absorption may further be aided by combining the use of hostile biophysical environments with the use of penetrating agents such as oleoresin capsicum or its constituents or moleculescontaining heterocyclic rings to which are attached hydrocarbon chains.

If a high or low pH environment is the hostile environment chosen, the preferred pH range is about between 3 to 11 pH.

### CLINICAL APPLICATIONS

### Example 1

In this example, a person with very cold fingers was provided with the above warming cream consisting of a delivery vehicle of penetrating cream, L-arginine hydrochloride (15% W/V), and sodium chloride (10% w/v). The surface temperature of the subject fingers of the left hand varied from21 0C to 24"C. The warming cream was applied by rubbing it into the skin. Surface temperatures of each finger were measured at 15 minute intervals for the initial hour. At the initial 15 minute interval following administration of the warming cream, an identifiable effect had begun to occur with surface temperatures of various fingers rising to26"C to 290C The maximal effect was reached by 45 minutes with surface temperatures of various fingers becoming31 0C to 34"C. The effect was sustained for at least 4 hours.

### Example 2

In this example, a person (female, age52) with very cold fingers was provided with the above warming cream consisting of a delivery vehicle of penetrating cream, L-arginine hydrochloride (12.5%), choline chloride (10%), magnesium chloride (5%) and sodium chloride (5%). The surface temperature of the subject fingers of the left hand varied from 21 to 24C". The warming cream was applied by rubbing it into the skin. Surface temperatures of each finger were measured at 15 minute intervals for the initial hour. At the initial 15 minute interval following administration of the warming cream, a noticeable effect had begun to occur with the surface temperatures or various fingers rising to between 26 to 29CO Maximal effect was reached by 45 minutes with surface temperatures of various fingers reaching 31 to 34C". The effect was sustained for at least 4 hours.

As illustrated by the examples, it can accordingly be seen that the present invention provides a method for administering a nitric oxide releasing substance in a delivery vehicle which when applied to cold, and often painful tissue, causes an increase in skin temperature through utilization of one of the body's own mechanisms for producing warmth. This effect is achieved by providing at the local site, the biochemical substrate from which the controlling substance, nitric oxide, is produced. Nitric oxide causes increases in local blood flow which results in warming.

## Claims

1. A composition for use as a medicament for topical application for increasing blood flow for warming cold or cool tissue, the composition comprising:
a nitric oxide releasing substance selected from the group consisting of L-arginine. L. arginine salts and alkyl esters of L-orginine, wherein the alkyl is selected from the group ethyl, methyl, propyl, isopropyl, butyl, isobutyl, t-butyl; and
a substance delivery carrier comprising a concentration of ionic salt, wherein the ionic salt has an ionic strength greater than two times the physiological ionic strength of blood.

2. The composition for use according to claim 1, **characterized in that** the ionic salt is selected from the group consisting of choline chloride preferably 0,25 % to 25% w/v thereof, sodium chloride, magnesium chloride and mixtures thereof.

3. The composition for use according to claim 1 or 2, **characterized in that** the nitric oxide releasing substance has a concentration of about between 0,25% to 25% w/v.

4. A composition according to any for use according to claims 1 to 3 comprising:
about 12,5% w/v L-arginine hydrochloride;
about 10.0% w/v choline chloride;
about 5% w/v sodium chloride;
about 5% w/v magnesium chloride; and,
a topical delivery vehicle.

5. The composition according to any for use according to claims 1 to 4, **characterized in that** the composition has a pH of about between 3 to 11 pH, and **in that** the substance delivery carrier is a hydrophobic delivery carrier.

6. The composition to any for use according to claims 1 to 5, **characterized in that** the substance delivery carrier contains L-arginine glutamate, preferably 0,25% to 25% w/v thereof.

7. The composition according to any for use according to claims 1 to 6. **characterized in that** the substance delivery carrier is selected from the group consisting of topical creams, topical liquids, topical lotions and topical ointments.

8. Use of composition according to any of claims 1 to 7 for making a medicament for warming cool or cold tissue

## Patentansprüche

1. Zusammensetzung zur Verwendung als äußerlich angewandtes Medikament zur wärmenden Durchblutungssteigerung eines kalten oder kühlen Gewebes, wobei die Zusammensetzung umfasst:
eine Stickoxid freisetzende Substanz ausgewählt aus der Gruppe bestehend aus L-Arginin, L-Arginin-Salze und Alkylester von L-Arginin, wobei das Alkyl aus der Gruppe von Ethyl, Methyl, Propyl, Isopropyl, Butyl, Isobutyl und T-Butyl ausgewählt ist; und
einen Substanzüberträger mit einem Gehalt an lonensalz, wobei das lonensalz eine lonenstärke hat, die größer als das Zweifache der physiologischen lonenstärke von Blut ist.

2. Zusammensetzung gemäß Verwendung nach Anspruch 1, **dadurch gekennzeichnet, dass** das lonensalz aus der Gruppe bestehend aus Cholin-Chlorid, davon vorzugsweise 0,25 % bis 25 % Raumgewicht. Kochsalz, Magnesium-Chlorid und Mischungen davon ausgewählt ist.

3. Zusammensetzung gemäß der Verwendung nach Anspruch 1 oder 2, **dadurch gekennzeichnet, dass** die Stickoxid freisetzende Substanz eine Konzentration zwischen etwa 0,25 % und 25 % Raumgewicht aufweist.

4. Zusammensetzung gemäß der Verwendung nach einem der Ansprüche 1 bis 3, **dadurch** gekenzeichnet, dass die Zusammensetzung umfasst:
etwa 12,5 % Raumgewicht L-Arginin Hydrochlorid;
etwa 10,0 % Raumgewicht Cholin-Chlorid;
etwa 5 % Raumgewicht Kochsalz:
etwa 5 % Raumgewicht Magnesium-Chlorid; und,
einen topischen Überträger.

5. Zusammensetzung gemäß der Verwendung nach einem der Ansprüche 1 bis 4, **dadurch gekennzeichnet, dass** die Zusammensetzung einen pH-Wert von etwa zwischen 3 und 11 aufweist und dass der Substanzüberträger ein hydrophober Überträger ist.

6. Zusammensetzung gemäß der Verwendung nach einem der Ansprüche 1 bis 5, **dadurch gekennzeichnet, dass** der Substanzüberträger ein L-Arginin Glutamat, davon vorzugsweise 0,25 % bis 25 % Raumgewicht, umfasst.

7. Zusammensetzung gemäß der Verwendung nach einem der Ansprüche 1 bis 6, **dadurch gekennzeichnet, dass** der Substanzüberträger aus der Gruppe bestehend aus topischen Cremen, topischen Flüssigkeiten, topischen Lotionen und topischen Salben ausgewählt ist.

8. Verwendung der Zusammensetzung nach einem der Ansprüche 1 bis 7 zur Herstellung eines Medikaments zum Wärmen eines kühlen oder kalten Gewebes.

## Revendications

1. Composition pour une utilisation en tant que médicament pour l'application topique afin d'augmenter le débit sanguin pour réchauffer un tissu froid ou refroidi, la composition comprenant :
une substance libérant de l'oxyde nitrique choisie dans le groupe constitué par la L.-arginine, les sels de L-arginine et les esters alkyliques de L-arginine, où l'alkyle est choisi dans le groupe constitué par éthyle, méthyle, propyle, isopropyle, butyle, isobutyle, t-butyle ; et
un véhicule de délivrance de substance, comprenant une concentration de sel ionique, lequel sel ionique a une force ionique supérieure à deux fois la force ionique physiologique du sang.

2. Composition pour une utilisation selon la revendication 1, **caractérisée en ce que** le sel ionique est choisi dans le groupe constitué par le chlorure de choline, de préférence 0,25 % à 25 % p/v de celui-ci, le chlorure de sodium, le chlorure de magnésium et les mélanges de ceux-ci.

3. Composition pour une utilisation selon la revendication 1 ou 2, **caractérisée en ce que** la substance libérant de l'oxyde nitrique a une concentration d'environ 0,25 % à 25 % p/v.

4. Composition pour une utilisation selon l'une quelconque des revendications 1 à 3, comprenant :
environ 12,5 % p/v de chlorhydrate de L-arginine ;
environ 10,0 % p/v de chlorure de choline ;
environ 5 % p/v de chlorure de sodium ;
environ 5 % p/v de chlorure de magnésium ; et
un véhicule de délivrance topique.

5. Composition pour une utilisation selon l'une quelconque des revendications 1 à 4, **caractérisée en ce que** la composition a un pH d'environ 3 à 11 et **en ce que** le véhicule de délivrance de substance est un véhicule de délivrance hydrophobe.

6. Composition pour une utilisation selon l'une quelconque des revendications 1 à 5, **caractérisée en ce que** le véhicule de délivrance de substance contient du glutamate de L-arginine, de préférence 0,25 % à 25 % p/v de celui-ci.

7. Composition pour une utilisation selon l'une quelconque des revendications 1 à 6, **caractérisée en ce que** le véhicule de délivrance de substance est choisi dans le groupe constitué par les crèmes topiques, les liquides topiques, les lotions topiques et les pommades topiques.

8. Utilisation d'une composition selon l'une quelconque des revendications 1 à 7, pour fabriquer un médicament pour réchauffer un tissu froid ou refroidi.
